# EUROPEAN PATENT APPLICATION

(11) **EP 4 324 847 A1**
(43) Date of publication of application: **21.02.2024**
(21) Application number: 22787516.8
(22) Date of filing: 12.04.2022
(51) Int. Cl.: C07K 14/55, A61K 38/20, A61P 37/02, A61P 35/00, A61P 29/00, A61P 25/28, A61P 31/00, G01N 33/68, G01N 33/574, G01N 33/569

(54) **INTERLEUKIN-2 MUTANT AND APPLICATION THEREOF**

(30) Priority: 13.04.2021 CN 202110387222
(71) Applicant: Suzhou Forlong Biotechnology Co., Ltd., Suzhou, Jiangsu 215002 (CN)
(72) Inventor: JI, En, Suzhou, Jiangsu 215002 (CN); WANG, Chunyu, Suzhou, Jiangsu 215002 (CN)
(74) Representative: V.O.
(86) International application number: PCT/CN2022/086279
(87) International publication number: WO 2022/218292

(57) **Abstract**

The present invention relates to an interleukin-2 (IL-2) mutant and an application thereof. Key sites were selected by means of computer-aided molecular design, and an IL-2 mutant phage display library was constructed based on synthetic biology techniques and combined with forward and reverse screening strategies to screen for a biased IL-2 mutant. Compared with wild-type IL-2, the IL-2 mutant of the present invention has reduced IL-2Rα receptor affinity and increased or equivalent IL-2Rβ receptor affinity, and can activate effector T cells, activate NK cells, and stimulate the release of interferon γ in a biased manner. Also provided in the present invention are a nucleic acid encoding the novel IL-2 mutant and a vector and a host cell comprising the nucleic acid, and further provided are a method for preparing the novel IL-2 mutant, a pharmaceutical combination comprising the novel IL-2 mutant, and a therapeutic use of the novel IL-2 mutant.

## Description

### Cross Reference to Related Applications

The present application claims the priority of Chinese Patent Application No. 2021103872223 filed with the China National Intellectual Property Administration on April 13, 2021, entitled "Interleukin-2 Mutant and Application Thereof', the entire content of which is incorporated into the present application by reference.

### Technical Field

The present invention belongs to the field of biotechnology and particularly relates to an interleukin-2 mutant, a preparation method therefor, a nucleic acid, plasmid, and host cell required for constructing the interleukin-2 mutant, a corresponding pharmaceutical composition, and an application thereof.

### Background Art

Interleukin-2 (IL-2) was discovered in 1976, and it was also called T cell growth factor (TCGF) at that time. It is mainly secreted by activated type I helper lymphocyte TH1 cells. In a precursor expressed by the interleukin gene, which consists of 153 residues, 20 amino acid residues at the N-terminal form a signal peptide, which is excised during secretion to produce mature secreted IL-2. IL-2 has four antiparallel, amphipathic α helices, which form a quaternary structure essential for its function.

IL-2 functions mainly via an IL-2 receptor on the membrane of an effector cell. The IL-2 receptor (IL-2R) has three subunits, P55, P75 and P64, named IL-2 receptor α (IL-2Rα, CD25), IL-2 receptor β (IL-2Rβ, CD122), and IL-2 receptor γ (IL-2Rγ, CD132), respectively. IL-2Rα binds to IL-2 with a low affinity (K_{D} of about 1 × 10⁻⁸ mol/L) and has no signaling function. IL-2Rγ does not bind to IL-2 *per se,* but shows a high affinity to IL-2 only after IL-2Rs α, β and γ combine (K_{D} of about 1 × 10⁻¹¹ mol/L), or β and γ combine (K_{D} of about 1 × 10⁻⁹ mol/L). The signaling function is related to β and γ, and more related to γ. IL-2Rα is mainly expressed on the surface of inhibitory regulatory T cells (Tregs) and some endothelial cells, while IL-2Rβ and IL-2Rγ are highly expressed on the surface of effector T cells (Teff) and NK cells. Quiescent effector T cells and NK cells are relatively insensitive to IL-2 because they have no IL-2Rα on the cell surface; however, Treg cells are more sensitive to low levels of IL-2 due to their high expression of IL-2Rα. Therefore, under normal circumstances, IL-2 will preferentially stimulate the proliferation of Treg cells. During the process of traditional tumor immunotherapies, in order to activate more Teff cells, it is necessary to use a high dose of IL-2, thus inevitably activating a large number of Tregs, leading to severe vascular leakage syndrome (VLS).

### Summary of the Invention

In order to solve the above technical problems, the present invention discloses a modification method for novel interleukin-2 mutants (IL-2 mutants) and a range of novel interleukin-2 mutants, and further discloses nucleic acid sequences encoding these interleukin-2 mutants and amino acid sequences thereof, vectors containing these nucleic acids, and host cells containing these vectors. The present invention further discloses a pharmaceutical composition of a novel interleukin-2 mutant. The IL-2 mutant and composition disclosed by the present invention can be used for the diagnosis and treatment of a disease. In other embodiments, these novel interleukin-2 mutants, nucleic acids, plasmids, and vectors are used for the diagnosis or treatment of a related disease such as an autoimmune disease or cancer.

In order to achieve the above object, the present invention provides the following technical solution:
An interleukin-2 mutant, comprising an amino acid sequence set-forth in SEQ ID NO: 1, wherein
X1 is selected from any amino acid of L, M, E, G, S, D, and Y;
X2 is any amino acid selected from D and E;
X3 is any amino acid selected from F, L, V, Y, and I;
X4 is any amino acid selected from K, H, G, Q, R, N, and S;
X5 is any amino acid selected from Y, F, and D;
X6 is any amino acid selected from F, A, Q, S, G, and L; and
X7 is any amino acid selected from C and A.

As a preferred embodiment of the interleukin-2 mutant described above, the interleukin-2 mutant comprises an amino acid sequence set forth in SEQ ID NO: 1, in which X1 is L, X2 is D, X3 is L, X4 is H, X5 is Y, X6 is F, and X7 is C; preferably, the interleukin-2 mutant comprises an amino acid sequence set forth in SEQ ID NO: 1, in which X1 is M, X2 is D, X3 is V, X4 is G, X5 is F, X6 is F, and X7 is C; more preferably, the interleukin-2 mutant comprises an amino acid sequence set forth in SEQ ID NO: 1, in which X1 is E, X2 is any amino acid selected from D and E, X3 is F, X4 is K, X5 is Y, X6 is any amino acid selected from A and G, and X7 is any amino acid selected from C and A.

As a preferred embodiment of the interleukin-2 mutant described above, the interleukin-2 mutant comprises an amino acid sequence set forth in SEQ ID NO: 1, in which X1 is E, X2 is D, X3 is F, X4 is K, X5 is Y, X6 is A, and X7 is C; preferably, the interleukin-2 mutant comprises an amino acid sequence set forth in SEQ ID NO: 1, in which X1 is E, X2 is E, X3 is F, X4 is K, X5 is Y, X6 is A, and X7 is C; more preferably, the interleukin-2 mutant comprises an amino acid sequence set forth in SEQ ID NO: 1, in which X1 is E, X2 is D, X3 is F, X4 is K, X5 is Y, X6 is G, and X7 is C; further preferably, the interleukin-2 mutant comprises an amino acid sequence set forth in SEQ ID NO: 1, in which X1 is E, X2 is D, X3 is F, X4 is K, X5 is Y, X6 is A, and X7 is A; more further preferably, the interleukin-2 mutant comprises an amino acid sequence set forth in SEQ ID NO: 1, in which X1 is E, X2 is D, X3 is F, X4 is K, X5 is Y, X6 is G, and X7 is A.

As a preferred embodiment of the interleukin-2 mutant described above, the interleukin-2 mutant comprises an amino acid sequence set forth in SEQ ID NO: 1, in which X1 is G, X2 is D, X3 is any amino acid selected from I, F, and Y, X4 is any amino acid selected from Q, N, and S, X5 is any amino acid selected from F, Y, and D, X6 is any amino acid selected from F and L, and X7 is any amino acid selected from C and A; preferably, the interleukin-2 mutant comprises an amino acid sequence set forth in SEQ ID NO: 1, in which X1 is G, X2 is D, X3 is I, X4 is Q, X5 is F, X6 is F, and X7 is C; more preferably, the interleukin-2 mutant comprises an amino acid sequence set forth in SEQ ID NO: 1, in which X1 is G, X2 is D, X3 is F, X4 is N, X5 is Y, X6 is F, and X7 is C; further preferably, the interleukin-2 mutant comprises an amino acid sequence set forth in SEQ ID NO: 1, in which X1 is G, X2 is D, X3 is Y, X4 is S, X5 is D, X6 is L, and X7 is C; more further preferably, the interleukin-2 mutant comprises an amino acid sequence set forth in SEQ ID NO: 1, in which X1 is G, X2 is D, X3 is F, X4 is N, X5 is Y, X6 is F, and X7 is A;
preferably, the interleukin-2 mutant comprises an amino acid sequence set forth in SEQ ID NO: 1, in which X1 is S, X2 is D, X3 is L, X4 is Q, X5 is Y, X6 is F, and X7 is C;
preferably, the interleukin-2 mutant comprises an amino acid sequence set forth in SEQ ID NO: 1, in which X1 is D, X2 is any amino acid selected from D and E, X3 is any amino acid selected from L and F, X4 is any amino acid selected from R and K, X5 is any amino acid selected from F and Y, X6 is any amino acid selected from F, Q, S, and A, and X7 is any amino acid selected from C and A;
preferably, the interleukin-2 mutant comprises an amino acid sequence set forth in SEQ ID NO: 1, in which X1 is D, X2 is D, X3 is L, X4 is R, X5 is F, X6 is F, and X7 is C;
preferably, the interleukin-2 mutant comprises an amino acid sequence set forth in SEQ ID NO: 1, in which X1 is D, X2 is E, X3 is F, X4 is K, X5 is Y, X6 is Q, and X7 is C;
preferably, the interleukin-2 mutant comprises an amino acid sequence set forth in SEQ ID NO: 1, in which X1 is D, X2 is E, X3 is F, X4 is K, X5 is Y, X6 is S, and X7 is C;
preferably, the interleukin-2 mutant comprises an amino acid sequence set forth in SEQ ID NO: 1, in which X1 is D, X2 is D, X3 is F, X4 is K, X5 is Y, X6 is A, and X7 is C;
preferably, the interleukin-2 mutant comprises an amino acid sequence set forth in SEQ ID NO: 1, in which X1 is D, X2 is E, X3 is F, X4 is K, X5 is Y, X6 is Q, and X7 is A;
preferably, the interleukin-2 mutant comprises an amino acid sequence set forth in SEQ ID NO: 1, in which X1 is D, X2 is E, X3 is F, X4 is K, X5 is Y, X6 is S, and X7 is A;
preferably, the interleukin-2 mutant comprises an amino acid sequence set forth in SEQ ID NO: 1, in which X1 is D, X2 is D, X3 is F, X4 is K, X5 is Y, X6 is A, and X7 is A; and
preferably, the interleukin-2 mutant comprises an amino acid sequence set forth in SEQ ID NO: 1, in which X1 is Y, X2 is D, X3 is L, X4 is G, X5 is Y, X6 is F, and X7 is C.
SEQ ID NO: 1 (the amino acid sequence of IL-2 mutant) 
APTSSSTKKTQLQLEHLLLDLQMILNGINNYKNPX1LTX2MLTX3X4FX5MPKKATE
LKHLQCLEEELKPLEEVLNX6AQSKNFHLRPRDLISNINVIVLELKGSETTFMCEYADETA
TIVEFLNRWITFX7QSIISTLT
SEQ ID NO: 2 (the amino acid sequence of mutant 21-C1) 
APTSSSTKKTQLQLEHLLLDLQMILNGINNYKNPLLTDMLTLHFYMPKKATELKHL
QCLEEELKPLEEVLNFAQSKNFHLRPRDLISNINVIVLELKGSETTFMCEYADETATIVEFL
NRWITFCQSIISTLT
SEQ ID NO:3 (the amino acid sequence of mutant 21-F9) 
APTSSSTKKTQLQLEHLLLDLQMILNGINNYKNPMLTDMLTVGFFMPKKATELKHL
QCLEEELKPLEEVLNFAQSKNFHLRPRDLISNINVIVLELKGSETTFMCEYADETATIVEFL
NRWITFCQSIISTLT
SEQ ID NO: 4 (the amino acid sequence of mutant 21-F10) 
APTSSSTKKTQLQLEHLLLDLQMILNGINNYKNPELTDMLTFKFYMPKKATELKHL
QCLEEELKPLEEVLNAAQSKNFHLRPRDLISNINVIVLELKGSETTFMCEYADETATIVEFL
NRWITFCQSIISTLT
SEQ ID NO: 5 (the amino acid sequence of mutant 22-A4) 
APTSSSTKKTQLQLEHLLLDLQMILNGINNYKNPGLTDMLTIQFFMPKKATELKHLQ
CLEEELKPLEEVLNFAQSKNFHLRPRDLISNINVIVLELKGSETTFMCEYADETATIVEFLN
RWITFCQSIISTLT
SEQ ID NO: 6 (the amino acid sequence of mutant 22-B2) 
APTSSSTKKTQLQLEHLLLDLQMILNGINNYKNPSLTDMLTQFYMPKKATELKHL
QCLEEELKPLEEVLNFAQSKNFHLRPRDLISNINVIVLELKGSETTFMCEYADETATIVEFL
NRWITFCQSIISTLT
SEQ ID NO: 7 (the amino acid sequence of mutant 22-D11) 
APTSSSTKKTQLQLEHLLLDLQMILNGINNYKNPDLTDMLTLRFFMPKKATELKHLQ
CLEEELKPLEEVLNFAQSKNFHLRPRDLISNINVIVLELKGSETTFMCEYADETATIVEFLN
RWITFCQSIISTLT
SEQ ID NO: 8 (the amino acid sequence of mutant 22-E8) 
APTSSSTKKTQLQLEHLLLDLQMILNGINNYKNPDLTEMLTFKFYMPKKATELKHL
QCLEEELKPLEEVLNQAQSKNFHLRPRDLISNINVIVLELKGSETTFMCEYADETATIVEFL
NRWITFCQSIISTLT
SEQ ID NO: 9 (the amino acid sequence of mutant 23-A5) 
APTSSSTKKTQLQLEHLLLDLQMILNGINNYKNPYLTDMLTLGFYMPKKATELKHL
QCLEEELKPLEEVLNFAQSKNFHLRPRDLISNINVIVLELKGSETTFMCEYADETATIVEFL
NRWITFCQSIISTLT
SEQ ID NO: 10 (the amino acid sequence of mutant 23-C8) 
APTSSSTKKTQLQLEHLLLDLQMILNGINNYKNPGLTDMLTFNFYMPKKATELKHL
QCLEEELKPLEEVLNFAQSKNFHLRPRDLISNINVIVLELKGSETTFMCEYADETATIVEFL
NRWITFCQSIISTLT
SEQ ID NO: 11 (the amino acid sequence of mutant 23-H9) 
APTSSSTKKTQLQLEHLLLDLQMILNGINNYKNPGLTDMLTYSFDMPKKATELKHL
QCLEEELKPLEEVLNLAQSKNFHLRPRDLISNINVIVLELKGSETTFMCEYADETATIVEFL
 NRWITFCQSIISTLT
SEQ ID NO: 12 (the amino acid sequence of mutant 24-C2) 
APTSSSTKKTQLQLEHLLLDLQMILNGINNYKNPDLTEMLTFKFYMPKKATELKHL
QCLEEELKPLEEVLNSAQSKNFHLRPRDLISNINVIVLELKGSETTFMCEYADETATIVEFL
NRWITFCQSIISTLT
SEQ ID NO: 13 (the amino acid sequence of mutant 24-C6) 
APTSSSTKKTQLQLEHLLLDLQMILNGINNYKNPELTEMLTFKFYMPKKATELKHLQ
CLEEELKPLEEVLNAAQSKNFHLRPRDLISNINVIVLELKGSETTFMCEYADETATIVEFLN
RWITFCQSIISTLT
SEQ ID NO: 14 (the amino acid sequence of mutant 24-D2) 
APTSSSTKKTQLQLEHLLLDLQMILNGINNYKNPELTDMLTFKFYMPKKATELKHL
QCLEEELKPLEEVLNGAQSKNFHLRPRDLISNINVIVLELKGSETTFMCEYADETATIVEFL
NRWITFCQSIISTLT
SEQ ID NO: 15 (the amino acid sequence of mutant 24-E4) 
APTSSSTKKTQLQLEHLLLDLQMILNGINNYKNPDLTDMLTFKFYMPKKATELKHL
QCLEEELKPLEEVLNAAQSKNFHLRPRDLISNINVIVLELKGSETTFMCEYADETATIVEFL
NRWITFCQSIISTLT
SEQ ID NO: 16 (the amino acid sequence of mutant 21-F10-C125A) 
APTSSSTKKTQLQLEHLLLDLQMILNGINNYKNPELTDMLTFKFYMPKKATELKHL
QCLEEELKPLEEVLNAAQSKNFHLRPRDLISNINVIVLELKGSETTFMCEYADETATIVEFL
NRWITFAQSIISTLT
SEQ ID NO: 17 (the amino acid sequence of mutant 22-E8-C125A) 
APTSSSTKKTQLQLEHLLLDLQMILNGINNYKNPDLTEMLTFKFYMPKKATELKHL
QCLEEELKPLEEVLNQAQSKNFHLRPRDLISNINVIVLELKGSETTFMCEYADETATIVEFL
NRWITFAQSIISTLT
SEQ ID NO: 18 (the amino acid sequence of mutant 23-C8-C125A) 
APTSSSTKKTQLQLEHLLLDLQMILNGINNYKNPGLTDMLTFNFYMPKKATELKHL
QCLEEELKPLEEVLNFAQSKNFHLRPRDLISNINVIVLELKGSETTFMCEYADETATIVEFL
NRWITFAQSIISTLT
SEQ ID NO: 19 (the amino acid sequence of mutant 24-C2-C125A) APTSSSTKKTQLQLEHLLLDLQMILNGINNYKNPDLTEMLTFKFYMPKKATELKHL 
QCLEEELKPLEEVLNSAQSKNFHLRPRDLISNINVIVLELKGSETTFMCEYADETATIVEFL
NRWITFAQSIISTLT
SEQ ID NO: 20 (the amino acid sequence of mutant 24-D2-C125A) 
APTSSSTKKTQLQLEHLLLDLQMILNGINNYKNPELTDMLTFKFYMPKKATELKHL
QCLEEELKPLEEVLNGAQSKNFHLRPRDLISNINVIVLELKGSETTFMCEYADETATIVEFL
NRWITFAQSIISTLT
SEQ ID NO: 21 (the amino acid sequence of mutant 24-E4-C125A)

APTSSSTKKTQLQLEHLLLDLQMILNGINNYKNPDLTDMLTFKFYMPKKATELKHL
QCLEEELKPLEEVLNAAQSKNFHLRPRDLISNINVIVLELKGSETTFMCEYADETATIVEFL
NRWITFAQSIISTLT

In a second aspect of the present invention, there is provided a nucleic acid molecule encoding the interleukin-2 mutant.

In a third aspect of the present invention, there is provided a plasmid containing the nucleic acid molecule, which is preferably operably linked to a regulatory sequence.

In a fourth aspect of the present invention, there are provided a host cell containing the plasmid, and a method for producing and, optionally, recovering the interleukin-2 mutant.

The host cell described in the present invention can be any prokaryotic cell or eukaryotic cell, including but not limit to bacterial cells (e.g., *Escherichia coli*), insect cells (e.g., using a baculovirus expression system), yeast or mammalian cells (e.g., CHO or BHK cell lines). Other suitable host cells are known to those skilled in the art. A preferred host cell is *Escherichia coli* cell, which has a short culture period and a relatively low production cost.

In a fifth aspect of the present invention, there is provided a pharmaceutical composition, wherein the pharmaceutical composition comprises a prophylactically or therapeutically effective dose of the interleukin-2 mutant according to any one of claims 1-4, the nucleic acid molecule according to claim 5, or the plasmid according to claim 6, and a physiologically or pharmaceutically acceptable carrier, and is prepared by means of mixing with an excipient or a stabilizer.

The pharmaceutical composition includes, but is not limited to, a freeze-dried dosage form, an aqueous solution dosage form, a liposome, a capsule dosage form, etc. The concentration of the interleukin-2 mutant of the present invention or the nucleic acid molecule or plasmid thereof may vary from about 0.1% to 100% (by weight).

In a sixth aspect of the present invention, there is provided the use of an effective dose of the interleukin-2 mutant according to any one of claims 1-4, the nucleic acid molecule according to claim 5, the plasmid according to claim 6, or the pharmaceutical composition according to claim 8 in the preparation of a medicament for the treatment of a biological material.

The present invention further provides a method for the diagnosis, prevention or treatment of a disease, comprising administering the interleukin-2 mutant, nucleic acid molecule, plasmid and pharmaceutical composition of the present invention to a subject. The disease is an autoimmune disease, an inflammatory disease, a neurodegenerative disease, a cancer, or a pathogen infection.

Preferably, the interleukin-2 mutant of the present invention can be used for treating cancer. The cancers described in the present invention include, but are not limited to, lymphoma, blastocytoma, sarcoma (including liposarcoma), neuroendocrine tumors, mesothelioma, schwannoma, meningioma, adenoma, melanoma, and aleukemic leukemia, or lymphatic malignancy. More specific examples of the above cancers include squamous cell carcinoma (e.g., squamous epithelial cell carcinoma), lung cancer, small cell lung cancer, non-small cell lung cancer, lung adenocarcinoma and lung squamous cell carcinoma, peritoneal cancer, hepatocellular carcinoma, gastric cancer, gastrointestinal cancer, pancreatic cancer, malignant glioma, cervical cancer, ovarian cancer, liver cancer, bladder cancer, hepatocellular carcinoma, breast cancer, colon cancer, rectal cancer, colorectal cancer, endometrial or uterine cancer, salivary gland cancer, renal cancer, prostate cancer, vulvar cancer, thyroid cancer, liver cancer, anal cancer, penile cancer, testicular cancer, esophageal cancer, bile duct tumor, head cancer, neck cancer, bone marrow stromal tumor, osteoclastoma, multiple myeloma, osteolytic bone cancers, central nervous system tumor, brain tumor (glioma, neuroblastoma, astrocytoma, medulloblastoma, ependymoma, and retinal neuroblastoma), nasopharyngeal carcinoma, basal cell carcinoma, cholangiocarcinoma, Kaposi's sarcoma, primary liver cancer or endometrial carcinoma, and vascular system tumor (angiosarcoma and hemagiopericytoma).

Preferably, the interleukin-2 mutant of the present invention can be used for treating a pathogen infection. The pathogens described in the present invention include, but are not limited to, bacteria, fungi, viruses, and parasites.

The interleukin-2 mutant of the present invention can be used for treating diseases including, but not limited to, congestive heart failure (CHF), vasculitis, rosacea, acne, eczema, myocarditis and other myocardial diseases, systemic lupus erythematosus, diabetes mellitus, spondylopathy, synovial fibroblast proliferation, bone loss, Paget's disease, disuse osteopenia, malnutrition, periodontal disease, familial spleen anemia, Langerhans cell histiocytosis, spinal cord injury, acute septic arthritis, osteomalacia, hypercortisolism, monostotic fibrous dysplasia, multiple osteofibrous dysplasia, periodontal reconstruction and fracture, sarcoidosis, bone metastasis/osteodynia treatment and humoral hypercalcemia of malignancy, ankylosing spondylitis and other spinal arthropathy, transplant rejection, virus infection, hematologic neoplasm, Hodgkin's lymphoma, and non-Hodgkin's lymphoma (Burkitt's lymphoma, small lymphocytic lymphoma/chronic lymphocytic leukemia, mycosis fungoides, mantle cell lymphoma, follicular lymphoma, diffuse giant B cell lymphoma, marginal zone lymphoma, hairy cell leukemia and lymphoplasmacytic leukemia), lymphocyte precursor cell tumor, B cell acute lymphoblastic aleukemic leukemia/lymphoma, T cell acute lymphoblastic aleukemic leukemia/lymphoma, thymoma, mature T and NK cell tumors, peripheral T cell aleukemic leukemia, mature T cell aleukemic leukemia/T cell lymphoma, large granular lymphocytic leukemia, Langerhans cell histiocytosis, myeloma of acute myelogenous granulocytic leukemia, mature acute myelogenous leukemia (AML), differentiated acute myelogenous leukemia, acute promyelocytic leukemia, acute myelomonocytic leukemia, acute monocytic leukemia, myelodysplastic syndrome, chronic myeloproliferative disease, chronic myelocytic leukemia, osteoporosis, hepatitis, HIV, AIDS, spinal arthritis, rheumatoid arthritis, inflammatory bowel disease (IBD), sepsis and septic shock, segmental ileitis, psoriasis, scleroderma, graft-versus-host disease (GVHD), allogenic islet graft rejection, hematological malignancies such as multiple myeloma (MM), myelodysplastic syndrome (MDS) and acute myelogenous leukemia (AML), and tumor-related inflammation, peripheral nerve injury, or demyelinating diseases.

In a seventh aspect of the present invention, there is provided a detection kit, comprising the interleukin-2 mutant, nucleic acid molecule or plasmid of the present invention. The detection kit is used for detecting pathogens and tumor cells. The pathogens include viruses, bacteria, fungi, parasitic infections, etc. The tumor cells include various benign tumor cells, malignant tumor cells (i.e., cancer cells), solid tumor cells, and hematogenous carcinoma cells.

Compared with the prior art, the beneficial effects of the present invention lie in that:
1. Based on the research of the biological function of IL-2, the present invention develops an IL-2 mutant which selectively agonizes IL-2Rβγ in a biased manner without binding to IL-2Rα, and can obtain the potential of reducing the toxicity of IL-2 treatment, such as avoiding VLS, and improving its clinical therapeutic efficacy, such as specifically activating Teff to kill tumors.
2. In the present invention, based on the synthetic biology technology, key sites of action of IL-2 against IL-2Rα are creatively selected by means of computer-aided molecular design, a phage display library of IL-2 site-directed random mutation is innovatively constructed, and forward and reverse screening strategies are combined to screen for a biased novel IL-2. Compared with wild-type IL-2, the screened novel IL-2 has a higher expression yield.
3. The novel IL-2 described in the present invention has a significantly reduced affinity to IL-2Rα, and instead of affecting the affinity of IL-2 to IL-2Rβ, the affinity of the novel IL-2 to IL-2Rβ is enhanced due to the change of protein conformation. In addition, the novel IL-2 can also significantly activate Teff and NK cells and the release of interferon γ. It shows the effectiveness of the possible anti-tumor treatment thereof and reduces side effects of the treatment in tumor patients.
4. The IL-2 mutant and composition described by the present invention can be used for the diagnosis and treatment of a disease. In other embodiments, these novel interleukin-2 mutants, nucleic acids, plasmids, and vectors are used for the diagnose or treatment of a related disease such as an autoimmune disease or cancer.

### Brief Description of the Drawings

FIG 1 shows the diversity of the construction of an IL-2 mutant phage library;
FIG 2 shows various screening protocols for novel IL-2 and polyclonal phage ELISA data;
FIG 2a shows the affinity enrichment of antibodies resulting from polyclonal ELISA detection and screening with IL-2Rα mutant;
FIG 2b shows the affinity enrichment of antibodies resulting from polyclonal ELISA detection and screening with IL-2Rβ mutant;
FIGs. 3a-3o sequentially show the affinity of the IL-2 mutants to IL-2Rα in Table 3, as detected by bio-layer interferometry (BLI);
FIGs. 4a-4o sequentially show the BLI-detected affinity of the IL-2 mutants to IL-2Rβ in Table 3, as detected by bio-layer interferometry (BLI);
FIG 5a shows the function of IL-2 mutants to activate Teff (CD4 positive T cells);
FIG 5b shows the function of IL-2 mutants to activate Teff (CD8 positive T cells);
FIG 6 shows the function of the IL-2 mutants to activate NK cells;
FIG 7 shows the function of the IL-2 mutants to stimulate the release of interferon γ; and
FIG 8 shows the affinity of the IL-2 mutant C125A site mutation proteins to IL-2Rα and IL-2Rβ, as detected by bio-layer interferometry (BLI).

### Detailed Description of Embodiments

In order to enable a person skilled in the art to better understand the solution of the present application, the technical solutions in embodiments of the present application will be described below clearly and completely in conjunction with examples. Obviously, the described embodiments are only some, rather than all, of the embodiments of the present application. Based on the examples of the present application, all other examples obtained by those of ordinary skill in the art without involving any inventive effort should belong to the scope of protection of the present application.

In order to understand the present invention more thoroughly, some definitions are listed below. The above definitions are intended to include grammatical equivalents.

As used herein, the term "IL-2" means that interleukin-2 is a cytokine of the chemokine family. It is a cytokine derived from a variety of cells (mainly produced by activated T cells) and also has multi-directional effects (mainly promoting the growth, proliferation and differentiation of lymphocytes). It plays an important role in immune responses, resistance to viral infections, etc., in the body, and can stimulate the proliferation of T cells that have been initiated by specific antigens or mitogenic factors; can activate T cells and promote the production of cytokines; stimulate NK cell proliferation, enhance the killing activity of NK cells and produce cytokines to induce LAK cell production; promote the proliferation of B cells and the secretion of antibodies; and activate macrophages.

As used herein, the term "mutant" means an individual in which mutation has occurred and which features a different phenotype from the wild type.

As used herein, the term "amino acid" means one of the 20 naturally occurring amino acids or any unnatural analogues, which can be located in a specified position. The term "protein" herein means at least two covalently linked amino acids, including proteins, polypeptides, oligopeptides, and peptides. A protein can be composed of naturally occurring amino acids and peptide bonds, or of synthetic peptide mimetic structures, which are called "analogues". Therefore, the term "amino acid" or "peptide residue" used herein means naturally occurring and synthetic amino acids. By way of example, for the purpose of the present invention, homophenylalanine, citrulline and norleucine are considered as amino acids for the purpose of the present invention. The term "amino acid" also includes imino acid residues such as proline and hydroxyproline. A side chain may be in the (R) or (S) configuration. In a preferred embodiment, an amino acid exists in the (S) or L-configuration. If non-naturally occurring side chains are used, non-amino acid substitutions can be used, for example, to prevent or delay *in vivo* degradation.

As used herein, the term "nucleic acid" means a polymer composed of nucleotide units (ribonucleotides, deoxyribonucleotides, related naturally occurring structural variants and their synthetic non-naturally occurring analogues) via phosphodiester bonds. Therefore, the term includes nucleotide polymers, in which nucleotides and bonds therebetween include non-naturally occurring synthetic analogues, such as but not limited to, thiophosphates, aminophosphates, methylphosphates, chiral methylphosphates, 2'-O-methylribonucleotides, peptide nucleic acids (PNAs), etc. For example, these polynucleotides can be synthesized using an automatic DNA synthesizer. The term "oligonucleotide" generally refers to a short polynucleotide, usually no more than about 50 nucleotides. It should be understood that when the nucleotide sequence is represented by a DNA sequence (i.e., A, T, G, and C), this also includes an RNA sequence in which "T" is replaced by "U" (i.e., A, U, G, and C).

Herein, conventional symbols are used to describe nucleotide sequences: the left-hand end of a single-stranded nucleotide sequence is the 5' end; and the left-hand direction of a double-stranded nucleotide sequence is called 5' direction. The direction in which 5' to 3' nucleotides are added to the newborn RNA transcript is called the transcription direction. A DNA strand with the same sequence as mRNA is called a coding strand.

As used herein, the term "plasmid" means a plasmid artificially constructed on the basis of a natural plasmid to adapt to laboratory operations. A nucleic acid molecule can be introduced into a host cell, thereby producing a transformed host cell. A vector may include a nucleic acid sequence that allows it to replicate in a host cell, such as origin of replication, and may also include one or more selectable marker genes and other genetic elements known in the art.

As used herein, the term "host cell", also called recipient cell, refers to a host cell that receives a foreign gene during transformation and transduction (infection).

As used herein, the term "pharmaceutically acceptable carrier" means a conventional pharmaceutically acceptable carrier. Remington's Pharmaceutical Sciences, EW Martin, Mack Publishing Co., Easton, Pa., 15th edition (1975), describes compositions and preparations suitable for drug delivery of one or more therapeutic compounds or molecules (such as one or more antibodies), and additional agents.

As used herein, the "diagnosis" of a disease refers to the diagnosis of a condition and the development thereof in a patient after examination. The "prevention" of a disease refers to inhibiting the complete development of the disease. The term "treatment" refers to therapeutic intervention to improve the signs or symptoms of a disease or a pathological condition after it begins to develop.

The term "administration" herein means selecting an appropriate route to introduce the substance into a subject. For example, if the selected route is intravenous, the composition is administered by introducing the substance into the vein of the subject.

The term "prophylactically/therapeutically effective dose" herein means an amount of a specific agent sufficient to achieve a desired effect in a subject treated with the agent. An exact dosage will depend on the purpose of treatment and can be determined by those skilled in the art by using well-known techniques. The dosage range can be 0.01-100 mg/kg body weight or more, such as 0.1, 1, 10, or 50 mg/kg body weight, preferably 1-10 mg/kg. As is well known in the art, in terms of the degradation of an antibody or Fc fusion, systemic or local drug delivery and new protease synthesis rate, as well as age, weight, general health, sex, diet, administration time, drug interaction, and the severity of the disease, adjustment may be necessary and can be determined by those skilled in the art by means of conventional experimental methods. Such agents include the monomeric Fc domain molecules described herein. In one non-limiting example, this may be the amount of an HIV-specific monomeric Fc domain (or HIV-specific CH3 domain molecule) for preventing, treating or ameliorating HIV infection. Ideally, a therapeutically effective amount of antibody is an amount sufficient to prevent, treat or ameliorate an infection or disease, such as that ascribable to HIV infection in a subject, without causing significant cytotoxic effects in the subject. A therapeutically effective amount of an agent for preventing, ameliorating and/or treating a subject will depend on the subject being treated, the type and severity of the indisposition, and the mode of administration of a therapeutic composition.

As used herein, the term "residue" means the position in a protein along with the amino acid identity associated therewith. For example, aspartic acid 297 (also known as Asn297 and N297) is a residue in human antibody IgG1.

As used herein, the term "encoding" means the inherent property of a specific nucleotide sequence in a polynucleotide, such as a gene, a cDNA or an mRNA, to serve as a template for the synthesis of other polymers and macromolecules by biological processes with either a defined nucleotide sequence or a defined amino acid sequence, and the biological properties resulting therefrom. Therefore, if the transcription and translation of mRNA produced from this gene result in a protein in cells or other biological systems, the gene encodes the protein. A coding strand, the nucleotide sequence of which is identical to mRNA and is usually provided in a sequence listing, and a non-coding strand, used as a transcription template, for a gene or a cDNA, can be referred to as encoding the protein or other product of that gene or cDNA. Unless otherwise specified, the term "nucleotide sequence encoding an amino acid sequence" includes all nucleotide sequences which are degenerate with each other and encode the same amino acid sequence. A nucleotide sequence that encodes a protein or an RNA may also include introns.

As used herein, the term "operably linked" means that when a first nucleic acid sequence is in a functional relationship with a second nucleic acid sequence, the first nucleic acid sequence is operably linked to the second nucleic acid sequence. For example, if a promoter affects the transcription or expression of a coding sequence, the promoter is operably linked to the coding sequence. Usually, the operably linked DNA sequence is continuous, and when necessary, two protein coding regions are linked in the same reading frame.

Unless otherwise specified, all the technical and scientific terms used herein have the same meaning as commonly understood by those of ordinary skill in the field to which the present disclosure belongs. Unless otherwise explicitly specified in the context, the singular terms "a", "an", and "the" include plural indicators. It is to be understood that all base sizes or amino acid sizes given for nucleic acids or polypeptides, as well as all molecular weights or molecular weight values, are approximate and provided for description. Although methods and materials similar or equivalent to those described herein can be used in the practice or testing of the present disclosure, suitable methods and materials are described below. The term "comprise" means "include". All publications, patent applications, patents and other references mentioned herein are incorporated by reference in their entirety. In case of conflict, the present description (including the explanation of terms) shall prevail. In addition, the materials, methods and examples are only illustrative and not restrictive.

The standard recombinant DNA technologies and molecular cloning technologies used in the examples are well known in the art (Ausubel, F. M. et al., Current Protocols in Molecular Biology, published by Greene Publishing Assoc. and Wiley-Interscience), and the materials and methods suitable for microbial growth are well known in the art. Main chemical and biochemical reagents are purchased from KAPA Biosystems, New England Biolabs, TransGen Biotech, Thermo Fisher Scientific, OMEGA bio-tek, etc.

The present invention discloses an IL-2 mutant comprising an amino acid sequence set forth in SEQ ID NO: 1, wherein
X1 is selected from any amino acid of L, M, E, G, S, D, and Y;
X2 is any amino acid selected from D and E;
X3 is any amino acid selected from F, L, V, Y, and I;
X4 is any amino acid selected from K, H, G, Q, R, N, and S;
X5 is any amino acid selected from Y, F, and D;
X6 is any amino acid selected from F, A, Q, S, G, and L; and
X7 is any amino acid selected from C and A.

The present invention discloses a nucleic acid molecule that encodes the IL-2 mutant;
the present invention discloses a plasmid containing the above nucleic acid molecule;
the present invention discloses a host cell containing the above plasmid;
the present invention discloses a pharmaceutical composition comprising a prophylactically or therapeutically effective dose of the above IL-2 mutant, the above nucleic acid molecule, or the above plasmid, and a physiologically or pharmaceutically acceptable carrier;
the present invention discloses the use of the above IL-2 mutant, the above nucleic acid molecule, the above plasmid, or the above pharmaceutical composition in the preparation of a medicament for the treatment of a biological material; and
the present invention discloses a detection kit, wherein the detection kit comprises the above IL-2 mutant, the above nucleic acid molecule, or the above plasmid; and preferably, the detection kit is used for detecting pathogens and tumor cells.

The present invention will be illustrated in detail below in conjunction with specific examples.

### Example 1

### Screening of IL-2 mutants

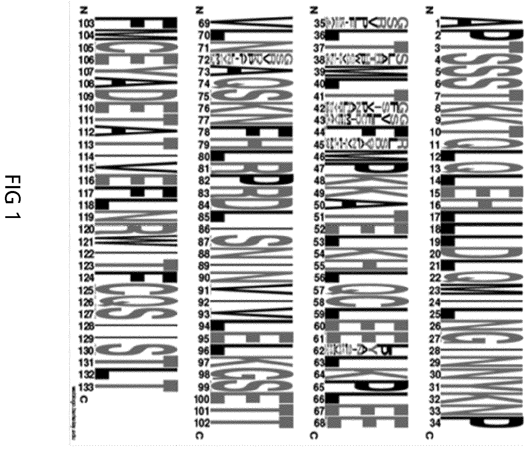

Key sites of action (K35, R38, F42, K43, Y45, E62, and L72) of IL-2 against IL-2Rα were selected by means of computer-aided molecular design, and these seven sites were randomly mutated, and an IL-2 mutant display library with a library capacity of 1.28 × 10⁹ was constructed by means of synthetic biology technology (as shown in FIG. 1) (see the literature PMID: 22518843 for the specific method).

Novel IL-2 mutants were screened against biotin-labeled soluble IL-2Rα and IL-2Rβ proteins by means of magnetic bead affinity panning method (for the specific method, referring to the literature PMID: 22518843).

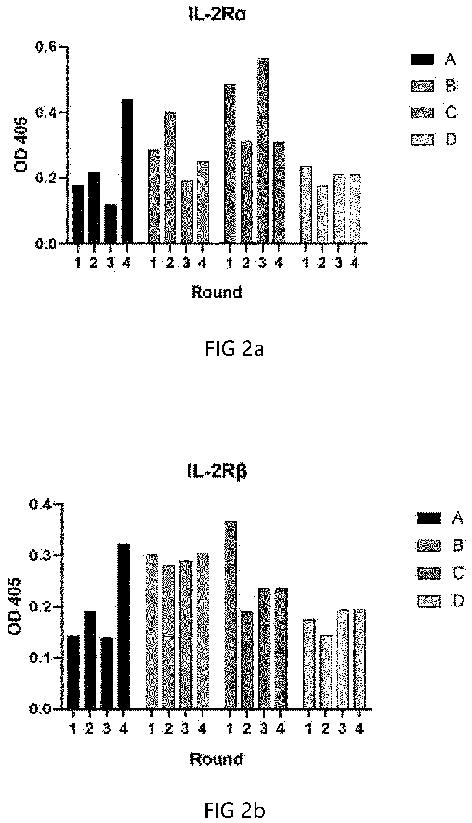

By means of a new strategy of double antigen alternate screening, the biotin-labeled soluble IL-2Rα and IL-2Rβ were successively allowed to specifically bind to streptavidin-coated magnetic beads (purchased from Invitrogen), and four protocols were used for forward and reverse screening (as shown in Table 1). After four rounds of double antigen alternate screening, polyclonal phage ELISA and monoclonal phage ELISA were further performed for screening (for the specific screening methods, referring to PMID: 28966056), a range of the finally screened novel IL-2 that bound IL-2Rβ with high affinity and bound IL-2Rα with low affinity or did not bind IL-2Rα were subjected to gene sequencing, and the enriched eligible novel IL-2 mutants were selected (as shown in FIGs. 2a and 2b). After sequencing, the key amino acid site mutations in the screened novel IL-2 mutants were analyzed (as shown in Table 2), and it was found that the three sites K35, R38 and L72 were the most important for binding to IL-2Rα, that is, the amino acid changes at these corresponding sites could completely destroy the binding to IL-2Rα.

**Table 1**

| A | B | C | D | ug |
|---|---|---|---|---|
| Rα | Rβ | Rα | Rβ | 1 |
| Rβ | Rα | Rα | Rβ | 1 |
| Rα | Rβ | Rβ | Rα | 1 |
| Rβ | Rα | Rβ | Rα | 1 |

**Table 2. IL-2 mutant sequence (WT-KRFKYEL)**

| Name | K35 | R38 | F42 | K43 | Y45 | E62 | L72 |
|---|---|---|---|---|---|---|---|
| 1.21-C1 | L | D | L | H | Y | E | F |
| 2.21-F9 | M | D | V | G | F | E | F |
| 3.21-F10 | E | D | F | K | Y | E | A |
| 4.22-A4 | G | D | I | Q | F | E | F |
| 5.22-B2 | S | D | L | Q | Y | E | F |
| 6.22-D11 | D | D | L | R | F | E | F |
| 7.22-E8 | D | E | F | K | Y | E | Q |
| 8.23-A5 | Y | D | L | G | Y | E | F |
| 9.23-C8 | G | D | F | N | Y | E | F |
| 10.23-H9 | G | D | Y | S | D | E | L |
| 11.24-C2 | D | E | F | K | Y | E | S |
| 12.24-C6 | E | E | F | K | Y | E | A |
| 13.24-D2 | E | D | F | K | Y | E | G |
| 14.24-E4 | D | D | F | K | Y | E | A |

### Example 2

The binding capacities of IL-2 mutants to IL-2Rα and IL-2Rβ were detected by bio-layer interferometry (BLI).

The binding capacities of the IL-2 mutants to IL-2Rα and IL-2Rβ were respectively detected by means of Anti-human IgG Fc Capture (AHC) probes (purchased from GE). The soluble expression and preparation of the IL-2 mutants were basically carried out according to the literature (YING, Tianlei et al., Journal of Biological Chemistry, 2012, 287 (23): 19399-19408).

The specific detection method involved diluting the IL-2 mutant protein by a factor of 3 (for the detection of the affinity to IL-2Rα, dilution was done from an initial concentration of 100 nM to 1.23 nM, and for the detection of the affinity to IL-2Rβ, dilution was done from an initial concentration of 1000 nM to 12.35 nM).

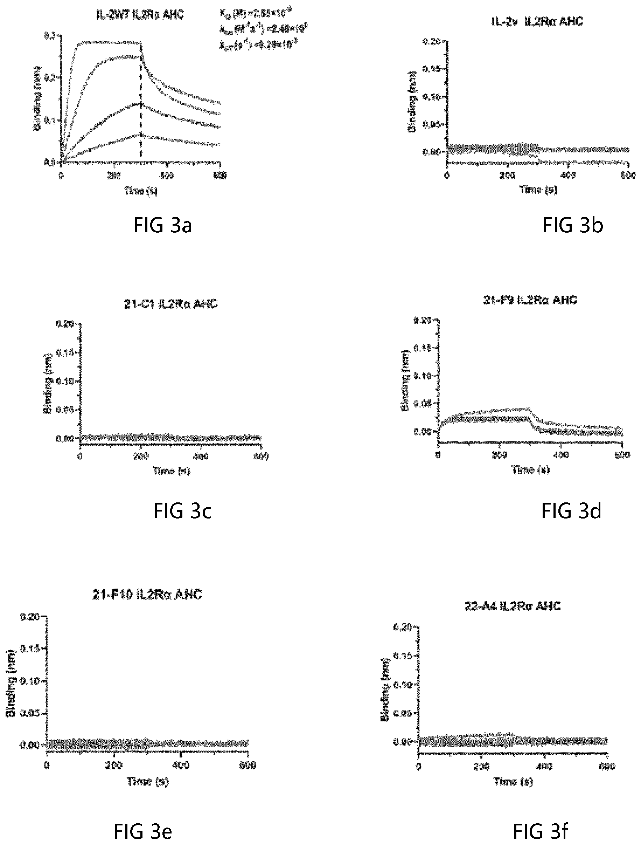

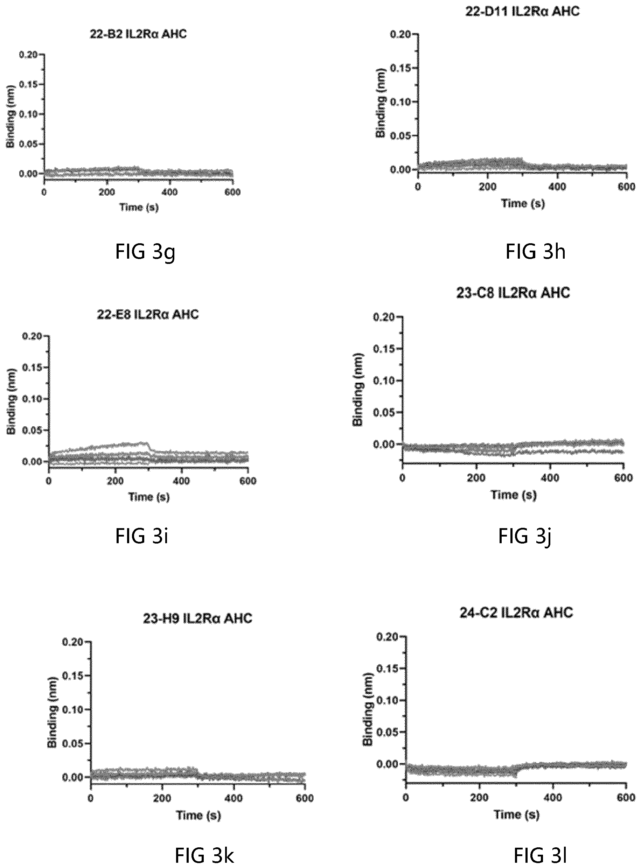

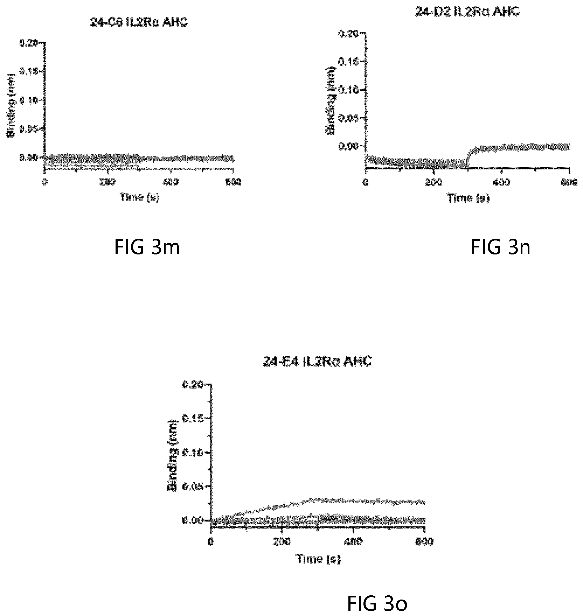

As shown in the results of FIGs. 3a-3o, due to the mutations at the key amino acid sites involved in binding to IL-2Rα, the IL-2 mutants essentially did not bind to IL-2Rα.

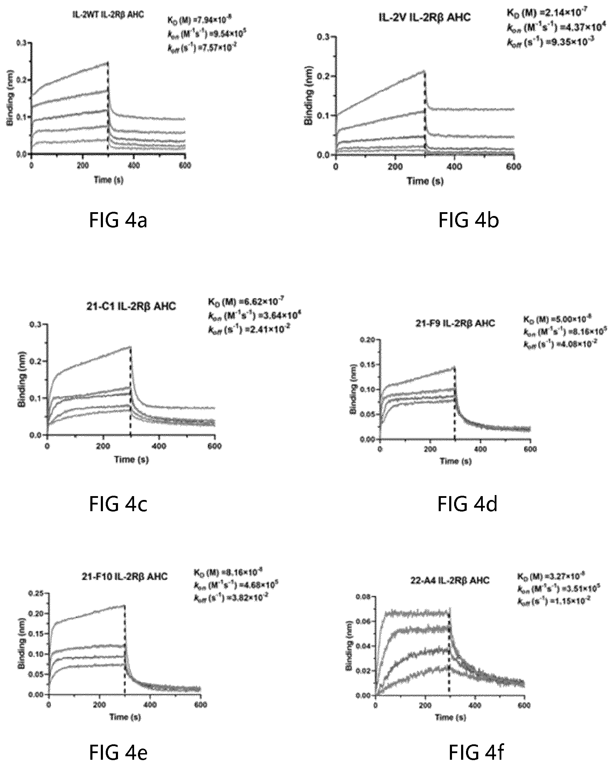

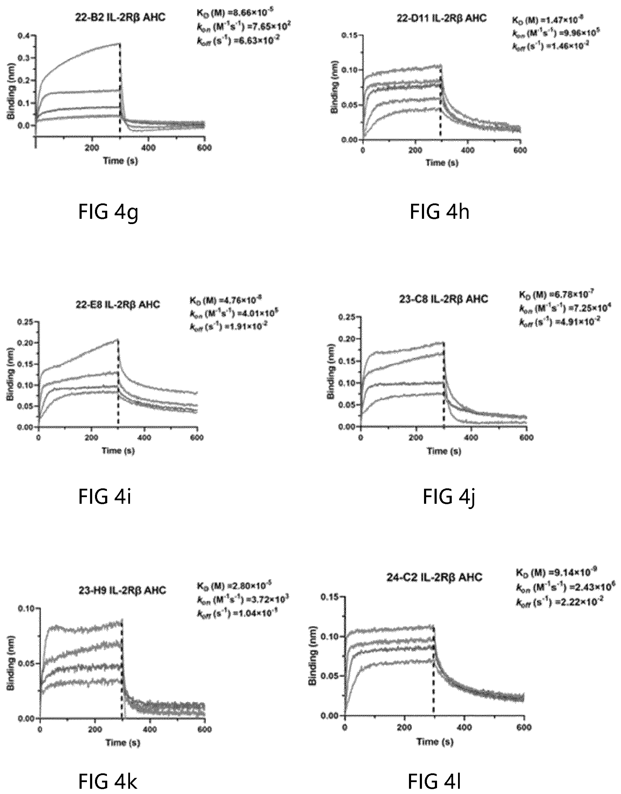

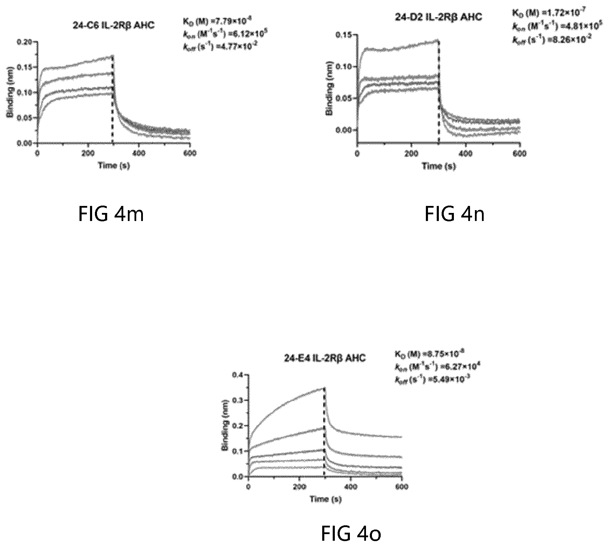

As shown in the results of FIGs. 4a-4o, due to the mutations at the key amino acid sites that did destroy the binding to IL-2Rβ or due to the conformational change, the IL-2 mutants maintained or even improved the capacity of binding to IL-2Rβ.

In an experiment carried out as described in the above assay, the affinity K_{D} values of fifteen IL-2 mutants expressed by Expi293 (purchased from Thermo Fisher Company) to their receptors were as shown in Table 3:

**Table 3. Affinity K_{D} values of IL-2 mutants to their receptors**

| Novel IL-2 | Affinity to IL-2Rα (M) | Affinity to IL-2Rβ (M) | Novel IL-2 | Affinity to IL-2Rα (M) | Affinity to IL-2Rβ (M) |
|---|---|---|---|---|---|
| IL-2WT | 2.55E-9 | 7.94E-8 | 23-C8 | N.B | 6.78E-7 |
| IL-2V | N.B | 2.14E-7 | 23-H9 | N.B | 2.80E-5 |
| 21-C1 | N.B | 6.62E-7 | 24-C2 | N.B | 9.14E-9 |
| 21-F9 | N.B | 5.00E-8 | 24-C6 | N.B | 2.48E-8 |
| 21-F10 | N.B | 8.16E-8 | 24-D2 | N.B | 1.72E-7 |
| 22-A4 | N.B | 3.27E-8 | 24-E4 | N.B | 8.75E-8 |
| 22-B2 | N.B | 8.66E-5 | | | |
| 22-D11 | N.B | 1.47E-8 | | | |
| 22-E8 | N.B | 4.76E-8 | | | |

As could be seen from the shown results: (1) the IL-2 mutants obtained from the phage library by means of double antigen alternate screening were detected to hardly bind to IL-2Rα; and (2) except for 22-B2 and 23-H9, the binding affinities of which to IL-2Rβ significantly decreased, 24-C2 had significantly increased binding affinity to IL-2Rβ, 21-C1, 23-C8, and 24-D2 bound to IL-2Rβ with affinities comparable to IL-2V, and for the remaining IL-2 mutants against IL-2Rβ, the affinity of IL-2 to IL-2Rβ stayed essentially unchanged.

### Example 3

### Experiment of the function of IL-2 mutants to stimulate effector cells (Teffs)

PBMC cells (Allcells Article No.: PB005F, 100 M packaged) were taken out of liquid nitrogen, quickly placed in a water bath at 37°C for about 1.5 min, and kept spinning continuously, without submerging the whole cryopreservation tube, and the PBMC cells were resuscitated.

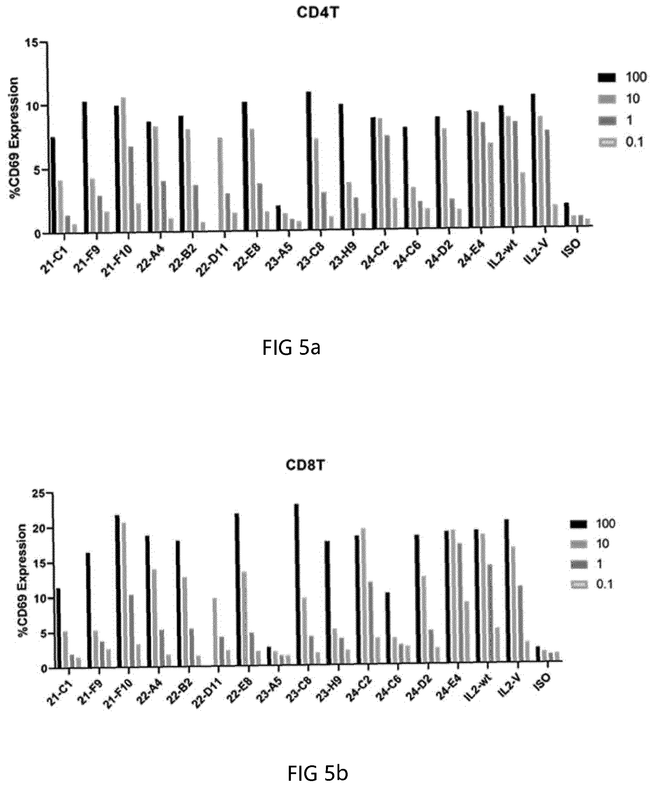

The cells were placed in a biosafety cabinet, and the outside of the cryopreservation tube was wiped with 75% ethanol. The PBMC cells were transferred with a 5 ml pipette to a 50 mL conical tube, in which hot RPMI (purchased from Gibco) with 10% FBS had been added in advance, and DMSO for cell freezing was removed by centrifugation. 20 mL of RPMI medium with 10% FBS was added to re-suspend the cells and was left to stand in a carbon dioxide incubator at 37°C for culturing overnight. The next day, RPMI medium containing 2% FBS was used to adjust the cell density to 2 × 10⁶/well, with a volume of 100 ul, and a flat-bottomed 96-well plate (Thermo edge 96 flat) was fully plated. Thereafter, the IL-2 mutant was diluted to four final concentrations, i.e., 100, 10, 1, and 0.1 nM, with medium 1640 containing 2% FBS, with 100 ul/well, and incubated for totally 48 h. Cells were collected by centrifugation and subjected to flow cytometry staining for CD3+CD4+/CD8+T (BioLegend's Cell Surface Flow Cytometry Protocol), and the collected data results were as shown in FIGs. 5a and 5b.

The IL-2 mutants 21-F10, 22-E8, 23-C8, 24-C2, 24-D2, 24-E4 had good effects on Teff activation, and these mutants were theoretically less immunogenic due to the fact that they had mutations at only three sites relative to wild-type IL-2.

### Example 4

### Experiment of the function of IL-2 mutants to stimulate NK cells.

The method for the resuscitation of PBMC cells was as above.

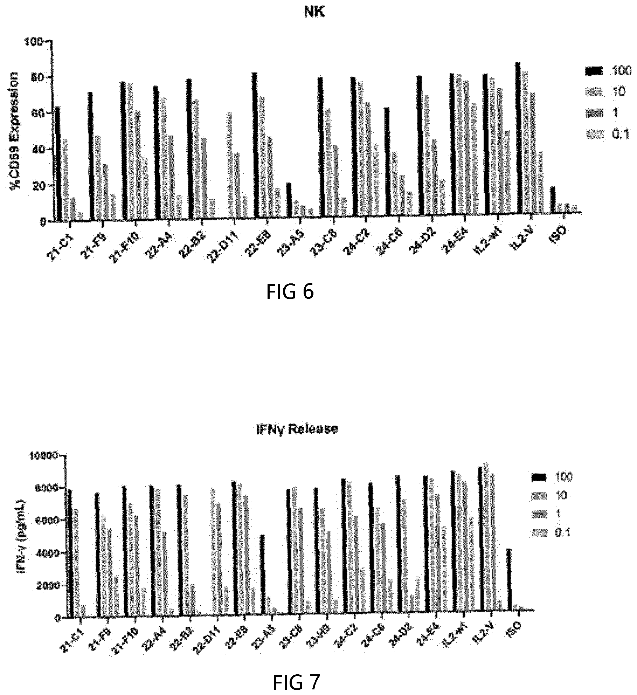

To detect the activity of stimulating NK cells, the novel IL-2 mutant was diluted to four final concentrations, i.e., 100, 10, 1, and 0.1 nM, with medium 1640 containing 2% FBS, with 100 ul/well, and co-incubated with the resuscitated PBMC cells for 48 h, cells were collected by centrifugation and subjected to flow cytometry staining for CD3-CD56+NK cells (BioLegend's Cell Surface Flow Cytometry Protocol). The collected data results were as shown in FIG 6.

Similarly to the effect of activating Teffs, the IL-2 mutants 21-F10, 22-E8, 23-C8, 24-C2, 24-D2, and 24-E4 also had better NK cell activation effects than the other IL-2 mutants.

### Example 5

### Experiment of the function of IL-2 mutants to stimulate interferon γ (IFNγ)

The method for the resuscitation of PBMC cells was as above.

The IL-2 mutants were diluted with medium 1640 containing 2% FBS to four final concentrations, i.e., 100, 10, 1, and 0.1 nM, with 100 ul/well, and co-incubated with the resuscitated PBMC cells for 48 h. 50 uL supernatant was taken to detect the release level of IFNγ (R&D DY285B), and the content of IFNγ was determined by ELISA method. As shown in FIG 7, the IL-2 mutants 21-F10, 22-E8, 23-C8, 24-C2, 24-D2, and 24-E4 had the most significant effect on stimulating the cells to secrete IFNγ.

### Example 6

### Construction of IL-2 mutant C125A site mutation and the binding capacity of the mutants to IL-2Rα and IL-2Rβ

The process of the construction of C125A site mutation was as follows: firstly, primers were designed according to the introductions of Beyotime QuickMutation^{™} Plus Gene Site-directed Mutation Kit (Article No.: D0208S): IL2new C125A-F: ACAGATGGATTACCTTTGCTCAGAGCATTATTAGCAC; and IL2new C125A-R: GTGCTAATAATGCTCTGAGCAAAGGTAATCCATCTGT. For the specific process, reference could be made to the introductions thereof.

The soluble expression and preparation of IL-2 mutant C125A mutation were basically carried out according to the literature (YING, Tianlei et al., Journal of Biological Chemistry, 2012, 287 (23): 19399-19408). The specific detection method involved using Anti-human IgG Fc Capture (AHC) probes (purchased from GE) to detect the binding capacity of the IL-2 mutant to IL-2Rα and IL-2Rβ, respectively, and diluting the IL-2 mutant C125A site mutation protein by a factor of 3 (for the detection of the affinity to IL-2Rα, dilution was done from an initial concentration of 100 nM to 1.23 nM, and for the detection of the affinity to IL-2Rβ, dilution was done from an initial concentration of 1000 nM to 12.35 nM).

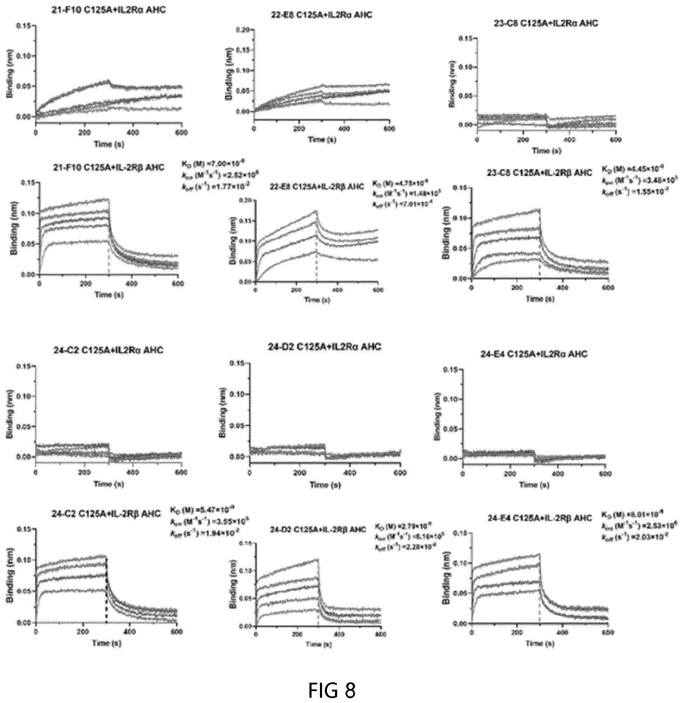

As shown in the results of FIG 8, due to the mutations at the key amino acid sites involved in binding to IL-2Rα, the IL-2 mutants essentially did not bind to IL-2Rα. As shown in the results of FIG 10, after C125A site mutation, the binding capacity of the IL-2 mutant to IL-2Rβ was essentially slightly superior to that of the IL-2 mutant without mutated C125 site, possibly because the mutation at C125 site increased the stability of the protein, so the binding thereof to IL-2Rβ would be stronger.

The above description of the disclosed examples enables those skilled in the art to implement or use the present invention. The above description is only preferred embodiments of the present invention, and it should be pointed out that those skilled in the art can also make several improvements and supplements without departing from the principles of the present invention, and these improvements and supplements should also be regarded as falling within the scope of protection of the present invention. Many modifications to these examples will be obvious to those skilled in the art, and the general principles defined herein can be implemented in other examples without departing from the spirit or scope of the present invention. Therefore, the present invention is not to be limited to the examples shown herein but is to be accorded the widest scope consistent with the principles and novel features disclosed herein.

## Claims

1. An interleukin-2 mutant, **characterized in that** the interleukin-2 mutant comprises an amino acid sequence set forth in SEQ ID NO: 1, wherein
X1 is selected from any amino acid of L, M, E, G, S, D, and Y;
X2 is any amino acid selected from D and E;
X3 is any amino acid selected from F, L, V, Y, and I;
X4 is any amino acid selected from K, H, G, Q, R, N, and S;
X5 is any amino acid selected from Y, F, and D;
X6 is any amino acid selected from F, A, Q, S, G, and L; and
X7 is any amino acid selected from C and A.

2. The interleukin-2 mutant according to claim 1, **characterized in that** the interleukin-2 mutant comprises an amino acid sequence set forth in SEQ ID NO: 1, in which X1 is L, X2 is D, X3 is L, X4 is H, X5 is Y, X6 is F, and X7 is C; preferably, the interleukin-2 mutant comprises an amino acid sequence set forth in SEQ ID NO: 1, in which X1 is M, X2 is D, X3 is V, X4 is G, X5 is F, X6 is F, and X7 is C; more preferably, the interleukin-2 mutant comprises an amino acid sequence set forth in SEQ ID NO: 1, in which X1 is E, X2 is any amino acid selected from D and E, X3 is F, X4 is K, X5 is Y, X6 is any amino acid selected from A and G, and X7 is any amino acid selected from C and A.

3. The interleukin-2 mutant according to claim 1, **characterized in that** the interleukin-2 mutant comprises an amino acid sequence set forth in SEQ ID NO: 1, in which X1 is E, X2 is D, X3 is F, X4 is K, X5 is Y, X6 is A, and X7 is C; preferably, the interleukin-2 mutant comprises an amino acid sequence set forth in SEQ ID NO: 1, in which X1 is E, X2 is E, X3 is F, X4 is K, X5 is Y, X6 is A, and X7 is C; more preferably, the interleukin-2 mutant comprises an amino acid sequence set forth in SEQ ID NO: 1, in which X1 is E, X2 is D, X3 is F, X4 is K, X5 is Y, X6 is G, and X7 is C; further preferably, the interleukin-2 mutant comprises an amino acid sequence set forth in SEQ ID NO: 1, in which X1 is E, X2 is D, X3 is F, X4 is K, X5 is Y, X6 is A, and X7 is A; more further preferably, the interleukin-2 mutant comprises an amino acid sequence set forth in SEQ ID NO: 1, in which X1 is E, X2 is D, X3 is F, X4 is K, X5 is Y, X6 is G, and X7 is A.

4. The interleukin-2 mutant according to claim 1, **characterized in that** the interleukin-2 mutant comprises an amino acid sequence set forth in SEQ ID NO: 1, in which X1 is G, X2 is D, X3 is any amino acid selected from I, F, and Y, X4 is any amino acid selected from Q, N, and S, X5 is any amino acid selected from F, Y, and D, X6 is any amino acid selected from F and L, and X7 is any amino acid selected from C and A; preferably, the interleukin-2 mutant comprises an amino acid sequence set forth in SEQ ID NO: 1, in which X1 is G, X2 is D, X3 is I, X4 is Q, X5 is F, X6 is F, and X7 is C; more preferably, the interleukin-2 mutant comprises an amino acid sequence set forth in SEQ ID NO: 1, in which X1 is G, X2 is D, X3 is F, X4 is N, X5 is Y, X6 is F, and X7 is C; further preferably, the interleukin-2 mutant comprises an amino acid sequence set forth in SEQ ID NO: 1, in which X1 is G, X2 is D, X3 is Y, X4 is S, X5 is D, X6 is L, and X7 is C; more further preferably, the interleukin-2 mutant comprises an amino acid sequence set forth in SEQ ID NO: 1, in which X1 is G, X2 is D, X3 is F, X4 is N, X5 is Y, X6 is F, and X7 is A;
preferably, the interleukin-2 mutant comprises an amino acid sequence set forth in SEQ ID NO: 1, in which X1 is S, X2 is D, X3 is L, X4 is Q, X5 is Y, X6 is F, and X7 is C;
preferably, the interleukin-2 mutant comprises an amino acid sequence set forth in SEQ ID NO: 1, in which X1 is D, X2 is any amino acid selected from D and E, X3 is any amino acid selected from L and F, X4 is any amino acid selected from R and K, X5 is any amino acid selected from F and Y, X6 is any amino acid selected from F, Q, S, and A, and X7 is any amino acid selected from C and A;
preferably, the interleukin-2 mutant comprises an amino acid sequence set forth in SEQ ID NO: 1, in which X1 is D, X2 is D, X3 is L, X4 is R, X5 is F, X6 is F, and X7 is C;
preferably, the interleukin-2 mutant comprises an amino acid sequence set forth in SEQ ID NO: 1, in which X1 is D, X2 is E, X3 is F, X4 is K, X5 is Y, X6 is Q, and X7 is C;
preferably, the interleukin-2 mutant comprises an amino acid sequence set forth in SEQ ID NO: 1, in which X1 is D, X2 is E, X3 is F, X4 is K, X5 is Y, X6 is S, and X7 is C;
preferably, the interleukin-2 mutant comprises an amino acid sequence set forth in SEQ ID NO: 1, in which X1 is D, X2 is D, X3 is F, X4 is K, X5 is Y, X6 is A, and X7 is C;
preferably, the interleukin-2 mutant comprises an amino acid sequence set forth in SEQ ID NO: 1, in which X1 is D, X2 is E, X3 is F, X4 is K, X5 is Y, X6 is Q, and X7 is A;
preferably, the interleukin-2 mutant comprises an amino acid sequence set forth in SEQ ID NO: 1, in which X1 is D, X2 is E, X3 is F, X4 is K, X5 is Y, X6 is S, and X7 is A;
preferably, the interleukin-2 mutant comprises an amino acid sequence set forth in SEQ ID NO: 1, in which X1 is D, X2 is D, X3 is F, X4 is K, X5 is Y, X6 is A, and X7 is A; and
preferably, the interleukin-2 mutant comprises an amino acid sequence set forth in SEQ ID NO: 1, in which X1 is Y, X2 is D, X3 is L, X4 is G, X5 is Y, X6 is F, and X7 is C.

5. A nucleic acid molecule, **characterized by** encoding the interleukin-2 mutant according to any one of claims 1-4.

6. A plasmid, **characterized by** comprising the nucleic acid molecule according to claim 5.

7. A host cell, **characterized by** comprising the plasmid according to claim 6.

8. A pharmaceutical composition, **characterized by** comprising a prophylactically or therapeutically effective dose of the interleukin-2 mutant according to any one of claims 1-4, the nucleic acid molecule according to claim 5, or the plasmid according to claim 6, and a physiologically or pharmaceutically acceptable carrier.

9. Use of an effective dose of the interleukin-2 mutant according to any one of claims 1-4, the nucleic acid molecule according to claim 5, the plasmid according to claim 6, or the pharmaceutical composition according to claim 8 in the preparation of a medicament for the treatment of a biological material.

10. A detection kit, **characterized in that** the detection kit comprises the interleukin-2 mutant according to any one of claims 1-4, the nucleic acid molecule according to claim 5, or the plasmid according to claim 6; and preferably, the detection kit is used for detecting pathogens and tumor cells.
